# EUROPEAN PATENT SPECIFICATION

(11) **EP 1 416 275 B1**
(45) Date of publication and mention of the grant of the patent: **26.08.2009**
(21) Application number: 03396097.2
(22) Date of filing: 22.10.2003
(51) Int. Cl.: G01N 33/52, G01N 33/487, G01N 21/78, G01N 21/86

(54) **Control device for rapid test**
Kontrolleinrichtung für Schnelltest
Dispositiv de contrôle de test rapide

(30) Priority: 31.10.2002 FI 20021935
(43) Date of publication of application: 06.05.2004
(73) Proprietor: Oy Reagena Ltd, 70900 Toivala (FI)
(72) Inventor: Kuronen, Ilpo, 71520 Kaislaistenlahti (FI)
(74) Representative: Pitkänen, Hannu Alpo Antero

(56) References cited:
- WO-A-00/31538
- WO-A-96/38720
- WO-A-97/09620
- WO-A-98/22824
- US-A- 3 964 871
- US-A1- 2002 055 126
- US-B1- 6 319 466

## Description

The invention relates to a control device for facilitating the interpretation of the results obtained with rapid test in accordance with the preamble of claim 1.

Popularity of different rapid tests as analytic devices is based on their user-friendly procedures, which make them easily applicable to professional and non-professional use. Rapid tests in professional use are generally aimed at analyses of blood samples, whereas tests made from urine or saliva samples can also be used in so called field conditions by non-professional users. One commonly used rapid test for home-use is the pregnancy test, which can be bought from retail shops, among other things. Other widely used rapid tests are drug tests, and tests used for diagnosing infectious diseases.

The function of rapid tests is based on technologies described e.g. in patent publication WO 92/01226, in which method the substance to be measured or analyse is bound to a membrane by antibody or antigen, and from where it can be detected by using particle suspension, enzyme reaction or fluorescence. These techniques can be roughly divided into two types on the basis of their function: lateral-flow tests and flow-through tests. In both applications coloured particles, enzymes or fluorochromes can be used for signal formation. In addition, in both applications the result emerges on a membrane, which is usually made of nitrocellulose but which can also be made of other material, for example, polyvinyl difluoride (PVDF), nylon, cellulose, glass fibre or their mixtures.

A general problem related to the rapid tests is distinguishing the difference between the signal of positive and negative test because the interpretation of the test is based on the emergence of a figure which can be visually detected. In the user instructions of many rapid tests it is generally advised that if, as a result of the test, a clearly visible figure emerges, for example a line, the test is positive. If the figure is not visible, the test is negative. Thus, the interpretation of the result is dependent on the user's estimation on the intensity of the figure. Many rapid tests form a visually detectable figure, even though the sample analyzed in the test would in reality be in either analytical or clinical sense negative. This problem is emphasized in situations where maximum analytical sensitivity is required from the test, or if the test is meant to detect weak affinities between the binding substances. For example, tests manufactured for diagnosis of acute contagious diseases should be able to detect acute phase antibodies from body excrete. These antibodies usually possess weak affinity and, thus, are difficult to detect in rapid tests. In these cases, the performance of the test has to be tuned as sensitive as possible so that also the number of negative results interpreted as positive results increase, because as a test result, a faintly visible ghost line is formed which can be falsely interpreted as a positive result.

Reliability of the rapid tests can be increased by using actual control samples that are know to give a positive or negative result. However, these kind of control samples always require an analysis, and they can be difficult to realize by the user making the analysis because, for example, the use of blood derived samples requires that strict safety regulations are followed.

In order to improve the quality of rapid tests, a number of innovations have been made aimed at controlling the function of rapid tests. Patent publications WO9822824, WO9731268, US4920046 and US6057165 disclose control methods which aim at informing the user that the test has functioned properly. In US-patent application 2002/0055126 has been described a test device having a control line, with help of which the functioning of reagents is controlled. In WO 00/31538 has been presented a test strip with having control zones for particle reagent used in the test and controlling for functioning of the test. In WO 96/38720 has been shown a device or cassette, in which there are control means for showing flowing of a liquid. These methods and devices do not, however, give any information on the problem related to distinguishing positive and negative test result.

The purpose of this invention is to disclose a control device that remove the problems related to the presently known methods and control devices. Moreover, the purpose of the invention is to disclose a control device which increase the reliability of the results obtained with rapid tests, and improve their functional value. Especially, the purpose of the invention is to provide a control device which makes the distinguishing of the positive and negative results easier than before.

The purpose of the invention is achieved with the method and control device characterised in the characteristic part of claim 1.

According to the invention, on the support is a figure, which is formed by dispensing coloured particle suspension on the support, the intensity of the figure corresponds to the intensity of the known sample, in which case the correct result is distinguished by comparing the intensity of the figure formed this way to the result obtained in the rapid test. Suspensions having different particle density are made of coloured or visible colour-producing suspension, which is used for signal formation in rapid tests, with which suspensions visually detectable figure is formed on a support used in control tests. With the help of the invention it is possible to distinguish the weak signals given by the rapid tests from true positive signals and background signals. The advantage of the invention is that when the result of the test is detectable either by visually or by reading optics as, for example, coloured line or some other kind of figure on the surface of a membrane, it can be compared with the previously analysed sample's corresponding line or other figure having the correct intensity. With the help of the invention the results of the rapid tests are made more reliable.

The intensity of a separate figure can be adjusted to correspond to the intensity formed by the positive and/or negative samples, and the intensity of the figure formed in this way can be compared to the result obtained from the rapid test, in which case the correct negative result is distinguished from the positive result, and/or correct positive result from the negative result or the background. Naturally, at least two figures can be formed on a support, from which other figure is for comparison of the positive and the other for comparison of the negative result.

In order to distinguish between the negative and positive signal, the intensity of the colour/colours of the figure/figures to be formed is/are adjusted to the control device by using known positive and negative samples, and the signals obtained from the rapid test of the analysed sample are controlled and distinguished with the help of figure or figures in the control device. The correct intensity of the figure/figures to be formed in the control device is obtained by real samples, by comparing the results of the test with the figure/ figures in the control device accurate results are obtained.

The figures in the control device according to the invention give adequate information on the intensity of the positive and negative signals in the rapid test device to the user of the control device. The invention facilitates the distinguishing of positive result from the negative result, and, thus, the results of the rapid tests are made more reliable, which, in turn, increases diagnostic functional value of rapid tests.

The figure used for distinguishing the signal is formed preferably on a membrane by using coloured particle suspension. The signal can be formed of particle suspension made of heavy metals such as gold or plastics, such as latex, or any other applicable material/materials, which particle suspension consists of particles having preferable particle size of 0,005 µm - 2 µm. The particle suspension can also be standardised spectrophotometrically by measuring optical density of suspensions at a proper wavelength for measuring suspension.

Preferably the support is a membrane made of any applicable light-coloured material such as nitrocellulose, cellulose, polyvinyldifluoride or nylon. The figure to be formed can be any visually detectable figure such as a line, plus- or minus-mark, or some other detectable figure.

The membrane having the figure is assembled to form a test strip, which can be used as such or assembled to a plastic holder, in which case a control device resembling the original analysis cassette is achieved. When it is a separate device, it can be easily used separately from the rapid test device, and the same control device can be used constantly. The control device that has a figure of a desired density is selected in such a way that the shape, width and intensity of the colour of figure in the control device is compared to the result of positive and negative samples acquired from the analytic device (rapid test). The control device having the figure giving the desired information is copied by using information acquired from spectrophotometer on the density of particle suspension, in which case all the prepared control devices contain exactly the correct figure.

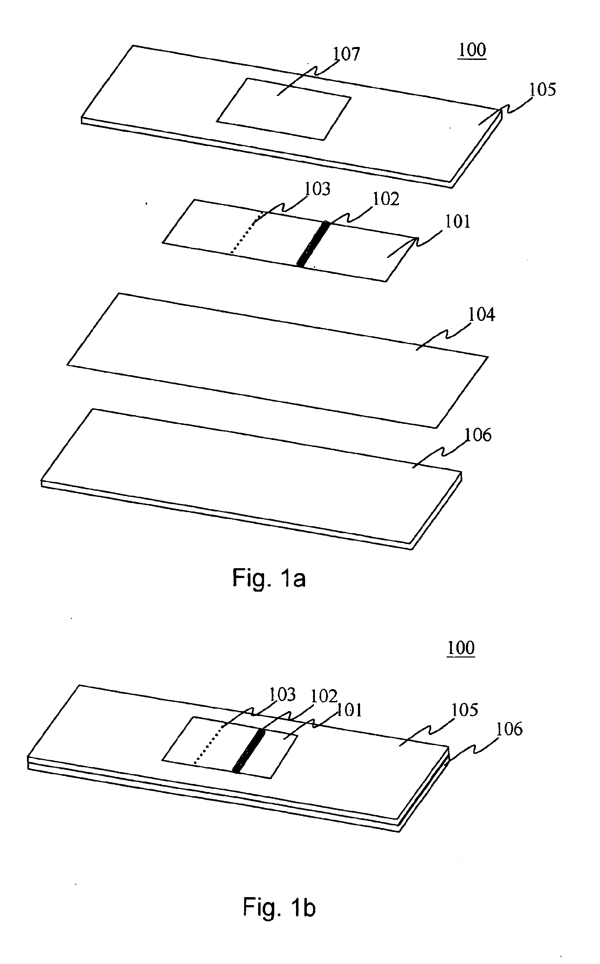

The invention is explained further in detail by referring to the following figures, in which
Fig. 1a presents a control device according to an embodiment of the invention and
Fig. 1b presents a control device according to Fig. 1 assembled.

Figures 1a and 1b present a control device according to an embodiment of the invention. The control device 100 is mainly strip-shaped and includes a frame 104 and support 101, which is preferably attached to the said frame. On the membrane 101 functioning as the support, figure 102, which resembles the control line in the analytic device 102, as well as the coloured line-shaped figure 103 has been made visible in the figures. The control device 100 may also consist of the cassette elements 105 and 106, between of which frame 104 and support 101 can be placed. Preferably the cassette element 105 includes an opening 107 wherein the figures 102 and 103 are visually detectable. The opening 107 may include a window such as, for example, a transparent plastic tape, which protects the support 101 from mechanical distress.

The frame 104, the cassette elements 105 and 106 and the window 107 can be prepared from, for example, plastic or any other applicable material. The membrane 101 can be prepared from, for example, nitrocellulose, cellulose, such as paper, polyvinyldifluoride, nylon or other light-coloured material. The shape of the figure 102 and 103 formed on a membrane 101 can be, in addition of a line, for example a plus- or minus-mark, or any other clearly detectable figure. The particle suspension can also be standardised spectrophotometrically by measuring optical density of the suspension at a proper wavelength for measuring suspension. The figure can be formed on a membrane 101 with, for this purpose made, liquid dispenser by dispensing pre-defined particle suspension on the said membrane. Alternatively any other methods for forming the desired figure can be used, such as printing technique, inkjet or laser printers. The used particle suspension is preferably coloured, and may be made of heavy metals such as gold, or plastics such as, for example latex, or any other particles having the particle size of 0,005-2 µm. The control device presented in figure 1 is used as separated device, and by means of it the results from the rapid tests can be easily observed and controlled.

### Example 1.

The following example describes a preparation of a control device in accordance with the invention, which example describes a level control for defining the signal level for nephropathia epidemica test, and in which coloured gold particles are used for signal formation. The example presented next is illustrated by reference to the enclosed table 1.

The rapid test manufactured by ERILAB OY (Kuopio, Finland) aimed at the diagnosis of acute nephropathia epidemica detects IgM-class antibodies in human blood samples, which in rapid test react to protein, manufactured by genetic engineering, originating from the Puumala virus. Because the test is for detection of real IgM-class antibodies as an early phase of the disease as possible, the test is vulnerable to formation of ghost lines with negative samples, that is, with samples which in reality are not samples from a patient suffering from nephropathia epidemica. The test uses coloured gold particles for the signal formation which have been labelled with the said IgM-class antibodies. In the test, coloured gold particles attach through real IgM-class antibodies on a membrane and form a red line. If there are no real antibodies, no red line should form. Frequently, however, the test shows a weak line with samples which are not from a patient suffering from nephropathia epidemica.
This phenomena can be due to variety of reasons such as cross-reacting antibodies, aggregation of the particle suspension, expired test, and so on. True samples from a patient suffering from nephropathia epidemica cause, however, a clearly detectable red line, and, thus, become interpreted correctly as positive samples. In order to distinguish a line which has been caused by a ghost line caused by a negative sample from the line caused by a real positive sample, the control test according to the invention was prepared as follows.

Suspensions having different particle densities was prepared from the red, gold particle suspension used for signal formation by diluting the original gold particle suspension with deionised water. The optical density of the suspensions can be measured, for example, by spectrophotometer at 520 nm wavelength in order to standardise the suspensions. Suspensions standardised this way are injected on a membrane used in nephropathia epidemica test in narrow lines, the width of which corresponds the width of lines of a nephropathia epidemica test. Membranes prepared this way are used to form control devices, in which the weak red line formed by the gold particles is visible at different intensities depending on the particle density of the suspension used. The control device, which includes the line of correct width, is then selected such that the line in the control device is compared with the results obtained from a sample giving the correct positive and negative ghost line. The control device, which includes a line which is weaker in intensity than the line in the positive sample, but at least as intense as the ghost line, which is created by negative sample in the nephropathia epidemica test, is selected for use as the control device or level control.

When analysing a group of known positive and negative samples, it became evident that in the results of the test there were a group of samples that were difficult to interpret neither positive or negative, since the intensity of the line of the samples was very weak. The user instructions advise that if the line is clearly visible, the test is positive. This caused a problem because clearly visible is not the same thing for different users, and the result may vary depending on the user. Interpretation of the same results with the help of a control devise gave a considerably better result since pre-defined intensity level of the line enabled the users to draw the right conclusions of the intensity of the line. Comparison was made by comparing the obtained result of analysis with the line in the control device. If the line obtained as a result of analysis was more visible (more intense) than the line in the control device, the result was positive. If the line was weaker or as weak as the line in the control device, the result was negative.

The enclosed table presents the results of the analysis obtained with and without the control device. The information in the table were collected from the series of routine analytics arrived in the clinical microbiological department of the University Hospital of Kuopio, the analysis of which has been known to cause problems in rapid tests previously. Progen Puumala IgM EIA (Progen GmbH, Germany) was used as the verification method of the samples in the clinical microbiological department of the University Hospital of Kuopio. In the point of view of practise, the deviation between the results is very significant, since when the interpretation became more explicit, it produced the correct result in case of several patients, which, in turn, enables the making of a right diagnosis.

### Example 2.

In the following, an alternative preparation method for a control device in accordance with the invention is presented, in which blue latex suspension is used as the signal formation

Many rapid tests use coloured latex particles for the signal formation, which can be of any colour. The use of blue coloured latex particles is more common than other colours, due to which they were chosen for this example. The signal former of the nephropathia epidemica test manufactured by ERILAB OY was replaced by the blue latex particle suspension (Banks Laboratories, USA), which was prepared by binding the same antibody, which was used in the case of above-mentioned gold particles, to the surface of the latex by adding the antibody to the particle suspension. Suspension prepared this way works similarly than the original gold particle suspension, but it produces a blue line instead of a red one.

The control device was prepared the same way as in the previous example, with the exception that in this example blue latex suspension was used. When the same series of samples were analysed with the tests producing a blue line, it became evident that depending on the function of the tests, the result was a series of weak test lines, the interpretation of which as negative or positive was difficult without a control device prepared this way, which illustrated the signal given by the support to the user.

Because the interpretation of any rapid test is based on detected figure or line formed or not formed depending on the sample, the previously presented examples apply universally indepedent from the analysed substance, manufacturer of the test, technique or colour formed. Without the illustrative example of the level of the background by the control device, the interpretation of the results may be difficult with samples, which contain a substance to be analysed with low content or low binding intensity.

The realisation and embodiments of the invention by way of examples have been presented above. It is evident for the person skilled in the art that the invention is not limited to the details of the above-presented embodiments, and that the invention can be realised in other form without deviating from the characteristics of the invention.

| **Sample ID** | **Reference result** | **Rapid test result Without control device** | **Rapid test result With control device** | **Comments** |
|---|---|---|---|---|
| 1 | Negative | Negative | Negative | No background |
| 2 | Negative | Uncertain | Negative | Background |
| 3 | Negative | Positive | Negative | Background |
| 4 | Positive | Positive | Positive | Clear result |
| 5 | Positive | Positive | Positive | Weak result |
| 6 | Positive | Positive | Positive | Clear result |
| 7 | Negative | Positive | Negative | Weak result |
| 8 | Negative | Negative | Negative | No background |
| 9 | Negative | Negative | Negative | No background |
| 10 | Negative | Negative | Negative | No background |
| 11 | Negative | Positive | Negative | Background |
| 12 | Negative | Uncertain | Negative | Background |
| 13 | Negative | Negative | Positive | Weak result |
| 14 | Positive | Positive | Positive | Clear result |
| 15 | Positive | Positive | Positive | Clear result |
| 16 | Positive | Positive | Positive | Clear result |
| 17 | Positive | Positive | Positive | Clear result |
| 18 | Positive | Positive | Negative | Weak result |
| 19 | Positive | Positive | Positive | Clear result |
| 20 | Negative | Positive | Negative | Background |
| 21 | Negative | Negative | Negative | Clear result |
| 22 | Negative | Negative | Negative | Clear result |
| 23 | Negative | Negative | Negative | Clear result |
| 24 | Negative | Negative | Negative | Clear result |
| 25 | Negative | Negative | Negative | Clear result |
| 26 | Negative | Negative | Negative | Clear result |
| 27 | Positive | Negative | Positive | Weak result |
| 28 | Negative | Negative | Negative | Clear result |
| 29 | Negative | Negative | Negative | Clear result |
| 30 | Negative | Negative | Negative | Clear result |

## Claims

1. Control device for facilitating the interpretation of the rapid test results, which control device includes a cassette (105, 106) including a support (101) with a figure (102, 103) on the support, **characterised in that** the cassette is a separate cassette from the rapid test device, the figure (102, 103) has been formed by dispensing coloured particle suspension, and that the intensity of this coloured particle suspension corresponds to the intensity of a known sample for a rapid test.

2. Control device according to claim 1, **characterised in that** the figure coloured particle suspension has been made of metal particles, such as gold, plastic particles, such as latex, having the particle size of 0,005-2,0 µm.

3. Control device according to claim 1 or 2, **characterised in that** the support (101) is a membrane prepared of light-coloured material, such as nitrocellulose, cellulose, polyvinyldifluoride or nylon.

## Patentansprüche

1. Kontrollvorrichtung zur Erleichterung der Interpretation der Schnelltestergebnisse, wobei die Steuervorrichtung eine Kassette (105, 106) enthält, die einen Träger (101) mit einer Zeichnung (102, 103) auf dem Träger enthält, **dadurch *gekennzeichnet*, dass** die Kassette eine von der Testvorrichtung getrennte Kassette ist, die Zeichnung (102, 103) durch Abgeben einer gefärbten Partikelsuspension gebildet wurde, und dass die Intensität dieser gefärbten Partikelsuspension der Intensität einer bekannten Probe für einen Schnelltest entspricht.

2. Kontrollvorrichtung nach Anspruch 1, **dadurch *gekennzeichnet*, dass** die gefärbte Partikelsuspension der Zeichnung aus Metallpartikeln, wie Gold, Kunststoffpartikeln, wie Latex, mit der Partikelgröße von 0,005 bis 2,0 µm besteht.

3. Kontrollvorrichtung nach Anspruch 1 oder 2, **dadurch *gekennzeichnet*, dass** der Träger (101) eine Membran ist, die aus einem hellfarbigen Material, wie Nitrozellulose, Zellulose, Polyvinyldifluorid oder Nylon, hergestellt ist.

## Revendications

1. Dispositif de contrôle destiné à faciliter l'interprétation des résultats de test rapide, ledit dispositif de contrôle comprenant une cassette (105, 106) qui inclut un support (101), avec une ligne (102, 103) sur le support, ***caractérisé* en ce que** la cassette est une cassette séparée du dispositif de test rapide, la ligne (102, 103) ayant été formée en appliquant une suspension de particules colorées, et **en ce que** l'intensité de cette suspension de particules colorées correspond à l'intensité d'un échantillon connu pour les tests rapides.

2. Dispositif de contrôle selon la revendication 1,
***caractérisé* en ce que** la suspension de particules colorées des chiffres est constituée de particules de métal, tel que l'or, de particules de plastique, tek que le latex, avec une taille de particules entre 0,005 et 2,0 µm.

3. Dispositif de contrôle selon la revendication 1 ou 2, ***caractérisé* en ce que** le support (101) est une membrane préparée à partir d'un matériau légèrement coloré, tel que la nitrocellulose, la cellulose, le difluorure de polyvinyle ou le nylon.
